# EUROPEAN PATENT APPLICATION

(11) **EP 2 208 722 A1**
(43) Date of publication of application: **21.07.2010**
(21) Application number: 08843193.7
(22) Date of filing: 01.10.2008
(51) Int. Cl.: C07C 303/16, B01J 31/22, C07C 269/06, C07C 271/18, C07C 271/22, C07C 309/38, C07D 213/06, C07D 263/26, C07B 53/00, C07B 61/00

(54) **PROCESS FOR PRODUCTION OF DISULFONIC ACID COMPOUND, ASYMMETRIC MANNICH CATALYST, PROCESS FOR PRODUCTION OF -AMINOCARBONYL DERIVATIVE, AND NOVEL DISULFONATE SALT**

(30) Priority: 24.10.2007 JP 2007276589; 24.10.2007 JP 2007276590
(71) Applicant: National University Corporation Nagoya University, Nagoya-shi, Aichi 464-8601 (JP)
(72) Inventor: ISHIHARA, Kazuaki, Nagoya-shi Aichi 464-8601 (JP); HATANO, Manabu, Nagoya-shi Aichi 464-8601 (JP); MAKI, Toshikatsu, Nagoya-shi Aichi 464-8601 (JP)
(74) Representative: Grünecker, Kinkeldey, Stockmair & Schwanhäusser Anwaltssozietät
(86) International application number: PCT/JP2008/067854
(87) International publication number: WO 2009/054240

(57) **Abstract**

Hexamethylphosphoramide (HMPA) was added to a reaction vessel containing (R)-1,1'-binaphthyl-2,2'-dithiol and potassium hydroxide. The vessel was purged with oxygen and stirred at 80°C for 5 days under 7 atmospheres of oxygen. After being cooled to room temperature, the reaction product was purified to yield potassium (R)-1,1'-binaphthyl-2,2'-disulfonate. The (R)-1,1'-binaphthyl-2,2'-disulfonic acid obtained from the disulfonate and 2,6-diphenylpyridine were stirred in acetonitrile, and then the solvent was evaporated under reduced pressure. Subsequently, magnesium sulfate and distilled CH₂Cl₂ were added to the reaction product, and the mixture was stirred at room temperature for 30 minutes. The resulting solution was cooled to 0°C. Benzaldehyde imine whose nitrogen is protected with Cbz and subsequently acetyl acetone were dropped into the solution over a period of 1 hour. The resulting mixture was further stirred at 0°C for 30 minutes. A corresponding β-aminocarbonyl derivative was thus produced with an yield of 91% and an enantiomeric excess of 90% ee.

## Description

### Technical Field

The present invention relates to a process for manufacturing a disulfonic acid compound, an asymmetric Mannich catalyst, a process for manufacturing a β-aminocarbonyl derivative, and a novel disulfonate.

### Background Art

It has been known that 1,1'-binaphthyl-2,2'-disulfonic acid is useful. For example, Patent Document 1 discloses that it can be used as an agent for introducing an asymmetric auxiliary group to an optically active amine. According to the document, when disulfonyl chloride derived from (S)-1,1'-binaphthyl-2,2'-disulfonic acid with thionyl chloride was allowed to react with racemic 1-phenylethylamine dissolved in acetonitrile, the NMR analysis of the reaction product, that is, a mixture of diastereoisomers of N-(1-phenylethyl)-1,1'-binaphthyl-2,2'-disulfonic acid amides, showed nonequivalent peaks derived from the proton at the α-position of the phenyl group of the amide portion of each diastereoisomer. Patent Document 1 describes a process for producing 1,1'-binaphthyl-2,2'-disulfonic acid in which a compound having SC(=O)NR₂ bonded to the 2- and 2'-positions of 1,1'-dinaphthyl is allowed to react with N-bromosuccinimide in the presence of a tertiary alcohol to derived a compound having -SO₂Br bonded to the 2- and 2'-positions, followed by hydrolysis.

It has been known that a β-aminocarbonyl derivative is produced by a Mannich reaction of an aldimine compound and a carbonyl compound. β-Aminocarbonyl derivatives are used in a variety of fields including the fields of pharmaceuticals and agricultural chemicals. A process has recently been proposed for producing an optically active β-aminocarbonyl derivative with a high enantiomeric excess (ee) by a Mannich reaction using an optically active phosphoric acid derivative derived from 1,1'-binaphthyl-2,2'-diol as a catalyst (see Non-Patent Document 1). More specifically, when the phosphoric acid derivative has unsubstituted naphthalene rings, the reaction product, optically active β-aminocarbonyl derivative, exhibits an enantiomeric excess as low as 12% ee. On the other hand, when the naphthalene rings of the phosphoric acid derivative have phenyl groups at the 3- and 3'-positions, the enantiomeric excess is increased to 56% ee. Also, when the phosphoric acid derivative has biphenyl groups at the 3- and 3'-positions, and when the phosphoric acid derivative has 4-(β-naphthyl)phenyl groups at the 3- and 3'-positions, the enantiomeric excesses are as high as 90% ee and 95% ee, respectively.
Patent Document 1: JP 2005-132815 A
Non-Patent Document 1: J. Am. Chem. Soc., vol. 126 (2004), No. 17, pp. 5356-5357

### Disclosure of Invention

Although Patent Document 1 describes a process for producing 1,1'-binaphthyl-2,2'-disulfonic acid, this process includes bromination, and is accordingly not necessarily advantageous. In addition, Patent Document 1 does not describe details of the process, such as the amounts of the reagents used for the reaction, the reaction conditions and the yield. It is therefore not easy for even those skilled in the art to replicate the same process.

There are not many known models of the synthesis of disulfonic acid by oxidizing dithiol in general organic synthesis. Possible oxidation reactions include, for example, chromic acid oxidation, periodic acid oxidation, iodine oxidation, oxidation using an aqueous solution of Oxone (trade name of a product available from Du Pont) (2KHSO₅·K₂SO₄·KHSO₄). However, the inventors of the present invention do not know any successful model of disulfonic acid synthesis by those oxidation reactions of dithiol. It has been reported about oxidation of dithiol that, for example, disulfide was produced with an yield of 90% by oxidizing dithiol with iodine (J. Org. Chem., vol. 58, pp. 1748-1750 (1993)). It has also been reported that monothiol was converted to monosulfonic acid by oxygen oxidation, and this reaction produced disulfide as a by-product (Tetrahedron, vol. 21, pp. 2271-2280 (1965)). The above cases seems to suggest that when thiol groups are present at the sterically adjacent 2- and 2'-positions of a binaphthyl skeleton as in 1,1'-binaphthyl-2,2'-dithiol, there is a high possibility that the two sulfur atoms at the 2- and 2'-positions are bonded to each other to form an intramolecular disulfide by oxidation. The present inventors attempted the oxidations of 1,1'-binaphthyl 2,2'-dithiol with chromic acid, periodic acid, and Oxone in practice. However, these oxidations hardly produced disulfonic acid. The present inventors assume that one of the reasons is the by-production of an intramolecular disulfide, but this has not been confirmed.

When a phosphoric acid derivative disclosed in the above-cited Non-Patent Document 1 is used as a catalyst of Mannich reaction, tuning may be performed to find the most suitable catalyst for the substrate (aldimine compound or carbonyl compound) of the Mannich reaction. For this tuning, many compounds must be tested by introducing various substituents to the 3- and 3'-positions of the naphthalene rings of the phosphoric acid derivative. Unfortunately, such introduction of substituents is not easy, and tuning is difficult accordingly.

The present invention is intended to solve the above issues. An object of the present invention is to produce an axially asymmetric, optically active 1,1'-biaryl-2,2'-disulfonic acid compound with a high yield. Another object of the invention is to provide an asymmetric Mannich catalyst that can produce an optically active β-aminocarbonyl derivative with a high enantiomeric excess, and that facilitates the tuning for the substrate, and a process for manufacturing a β-aminocarbonyl derivative using the same.

The present inventors added hexamethylphosphoramide (HMPA) to a reaction vessel containing optically active 1,1'-binaphthyl-2,2'-dithiol and potassium hydroxide, and performed oxidation with pressurized oxygen to produce the potassium salt of the corresponding disulfonic acid with a high yield. Thus, the inventors accomplished the invention. In the process for manufacturing a disulfonic acid compound according to the present invention, an axially asymmetric, optically active 1,1'-biaryl-2,2'-dithiol compound is oxidized with pressurized oxygen in the presence of a strong base to produce a salt of a corresponding optically active disulfonic acid compound.

According to the manufacturing process of disulfonic acid compound of the present invention, an optically active 1,1'-biaryl-2,2'-dithiol compound is oxidized with environmentally friendly oxygen. This process can produce a salt of 1,1'-diaryl-2,2'-disulfonic acid compound with a high yield while maintaining the optical activity. The resulting salt is purified, for example, through a column, and then quantitatively converted to a free form of the disulfonic acid compound through a cation exchange resin. The thus obtained disulfonic acid compound can be used in a variety of applications. For example, the disulfonic acid compound can be used as an agent for introducing an asymmetric auxiliary group to an optically active amine, as disclosed in Patent Document 1, or it may be converted to the ammonium salt to use as an asymmetric Mannich catalyst, as described later in Examples.

Furthermore, the present inventors found that an optically active β-amino-α-acylcarbonyl derivative can be produced with a high enantiomeric excess by a Mannich reaction between benzaldehyde imine whose nitrogen is protected and acetyl acetone in the presence of the mixture of optically active 1,1'-binaphthyl-2,2'-disulfonic acid and 2,6-diphenylpyridine in a molar ratio of 1:2, and thus accomplished the present invention. The asymmetric Mannich catalyst of the present invention is a mixture of an optically active 1,1'-binaphthyl-2,2'-disulfonic acid compound and 2,6-disubstituted pyridine (substituent is aryl or branched alkyl) in a molar ratio of 1:0.75 to 1:3.

In a process for manufacturing a β-aminocarbonyl derivative according to the present invention, an aldimine compound expressed by Ar-CH=NR¹ (Ar represents aryl, R' represents tert-butoxycarbonyl (Boc), benzyloxycarbonyl (Cbz) or 2,2,2-trichloroethoxycarbonyl (Troc)) and a carbonyl compound are subjected to a Mannich reaction in the presence of the asymmetric Mannich catalyst to yield an optically active β-aminocarbonyl derivative.

A novel disulfonate according to the present invention is a salt of an optically active 1,1'-binaphthyl-2,2'-disulfonic acid compound and 2,6-disubstituted pyridine (substituent is aryl or branched alkyl).

The use of the asymmetric Mannich catalyst of the present invention allows an asymmetric Mannich reaction to produce an optically active β-aminocarbonyl derivative with a high enantiomeric excess. The use of the catalyst allows easy tuning for the aldimine compound and the carbonyl compound, which are reaction substrates. More specifically, since catalysts having various structures can be obtained by merely mixing an optically active 1,1'-binaphthyl-2,2'-disulfonic acid compound and various types of 2,6-disubstituted pyridine, catalysts having different structures can be prepared more easily than catalysts having various structures produced by introducing substituents to the naphthalene rings as in Non-Patent Document 1. Accordingly, the tuning can be facilitated which is performed to determine which structure the catalyst can have is suitable for the reaction substrate. This is a great advantage from an industrial viewpoint.

### Brief Description of Drawings

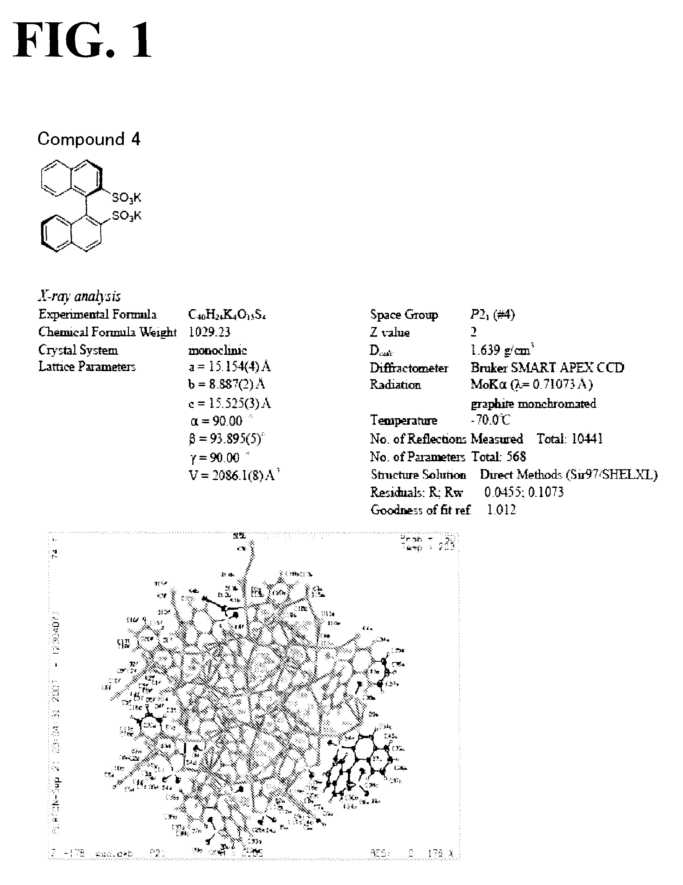

Fig. 1 shows the results of X-ray analysis of potassium 1,1'-binaphthyl-2,2'-disulfonate.

### Best Modes for Carrying Out the Invention

### [Process for manufacturing a disulfonic acid compound]

The optically active 1,1'-biaryl-2,2'-dithiol compound used in a process for manufacturing a disulfonic acid compound of the present invention may be in the R-form or the S-form according to the stereochemistry of axially asymmetric 1,1'-biaryls. In the present embodiment, an (R)-1,1'-biaryl-2,2'-dithiol compound may be used, or an (S)-1,1'-biaryl-2,2'-dithiol compound may be used. The biaryl of the 1,1'-biaryl-2,2'-dithiol compound has an aromatic hydrocarbon ring skeleton, and such biaryls include, for example, biphenyl, binaphthyl and biphenanthryl. Among those, preferred is binaphthyl. The biaryl may or may not be substituted. If the biaryl is substituted, for example, a substituent may be present at at least one of the 3-, 3'-, 6- and 6'-positions.

Preferably, the strong base used in the manufacturing process of the disulfonic acid compound of the present invention is, but not limited to, an alkali metal hydroxide, such as potassium hydroxide or sodium hydroxide. The strong base is preferably used in a proportion of more than 2 equivalents to the dithiol compound, more preferably 3 equivalents or more, and still more preferably 6 equivalents or more from the viewpoint of stably producing a disulfonate with a high repeatability with a high yield. Although the upper limit of the amount of the strong base to be used is not particularly limited, it is preferably 10 equivalents or less from an economical viewpoint.

The pressurized oxygen used in the manufacturing process of the disulfonic acid compound of the present invention is preferably, but not limited to, oxygen pressurized to 5 atmospheres or more from the viewpoint of stably producing a disulfonate with a high repeatability with a high yield. Oxygen pressurized to 7 atmospheres or more is particularly preferred. The use of such oxygen further increases the yield. The upper limit of the pressure of the pressurized oxygen is not particularly limited, but is preferably 20 atmospheres or less, more preferably 10 atmospheres or less, in view of safety and economy.

The manufacturing process of the disulfonic acid compound of the present invention may use a reaction solvent, if necessary. Polar aprotic solvents are preferably used. Exemplary polar aprotic solvents include phosphoric acid amide solvents such as hexamethylphosphoramide (HMPA), carboxylic acid amide solvents such as N,N-dimethylformamide, and tetraalkyl urea solvents such as 1,3-dimethyl-2-imidazolidinone, and HMPA is preferred.

The manufacturing process of the disulfonic acid compound of the present invention does not limit the reaction temperature. However, if the reaction temperature is too low, the oxidation reaction can become too slow, and if the reaction temperature is too high, a large amount of by-product can be produced. Accordingly, the reaction temperature is preferably set in the range of 50 to 100°C. The reaction time can be set to a period until the dithiol compound is consumed, or a period until the reaction stops, and it is set, in general, in the range of several hours to several days.

In the manufacturing process of the disulfonic acid compound of the present invention, the salt of the disulfonic acid compound prepared by oxidizing 1,1'-biaryl-2,2'-dithiol compound may be converted into a free form of the disulfonic acid compound through a cation exchange resin after purification through a column. For example, silica gel can be used as the column. The eluent for the column can be, for example, chloroform or methanol. For example, Amberlite (Rohm & Haas) can be used as the cation exchange resin, and water or methanol can be used as the eluent for the cation exchange resin.

### [Asymmetric Mannich Catalyst]

The optically active 1,1'-binaphthyl-2,2'-disulfonic acid compound used for the asymmetric Mannich catalyst of the present invention may be in the R-form or the S-form according to the stereochemistry of axially asymmetric 1,1'-binaphthyls (having a chiral axis). In the present embodiment, (R)-1,1'-binaphthyl-2,2'-disulfonic acid may be used, or (S)-1,1'-binaphthyl-2,2'-disulfonic acid may be used. The naphthalene rings of these compounds may or may not be substituted. The optically active 1,1'-binaphthyl-2,2'-disulfonic acid compound can be produced using, for example, optically active 1,1'-binaphthol as the starting material by converting its diol portion to dithiol, and then oxidizing the dithiol to disulfonic acid. Oxidizing agents containing a heavy metal, such as potassium dichromate can promote the oxidation of dithiol. However, such oxidizing agents have a high environmental load and a problem with waste disposal, and are accordingly not suitable. The present inventors attempted to oxidize the dithiol with generally used various oxidizing agents. As a result, most reactions did not proceed or many products were obtained except for the case of air oxidation (O₂ oxidation). The products could not be isolated. Accordingly, it is preferable that the dithiol be converted to disulfonic acid by air oxidation. Although the naphthalene rings of 1,1'-binaphthyl-2,2'-disulfonic acid may be substituted, it is preferable not substituted from the viewpoint of producing a catalyst having a structure as simple as possible.

The 2,6-disubstituted pyridine used for the asymmetric Mannich catalyst of the present invention has substituents at the 2- and 6-positions of pyridine. The substituent may be aryl or branched alkyl. The aryl group may be an unsubstituted aromatic hydrocarbon group, such as phenyl, naphthyl, phenanthryl or anthranil, or may have a substituent on the ring of the aromatic hydrocarbon group. Examples of the substituents on the ring of the aromatic hydrocarbon group include alkyl, alkenyl, cycloalkyl, aryl, and alkoxy. Exemplary alkyl groups include methyl, ethyl, n-propyl, isopropyl, n-butyl, isobutyl, sec-butyl, and tert-butyl. Exemplary alkenyl groups include vinyl, allyl, butenyl, and styryl. Exemplary cycloalkyl groups include cyclopentyl and cyclohexyl. Exemplary aryl groups include phenyl, biphenyl, naphthyl, binaphthyl, and anthryl.

Examples of 2,6-disubstituted pyridine whose substituents are aryl (2,6-diarylpyridine) include 2,6-diphenylpyridine; 2,6-di(4-alkylphenyl)pyridines, such as 2,6-di(4-methylphenyl)pyridine, 2,6-di(4-ethylphenyl)pyridine, 2,6-di(4-n-propylphenyl)pyridine, 2,6-di(4-isopropylphenyl)pyridine, 2,6-di(4-n-butylphenyl)pyridine, and 2,6-di(4-tert-butylphenyl)pyridine ; 2,6-di(4-arylphenyl)pyridines, such as 2,6-di(4-phenylphenyl)pyridine; 2,6-di(4-alkoxyphenyl)pyridines, such as 2,6-di(4-methoxyphenyl)pyridine and 2,6-di(4-ethoxyphenyl)pyridine; 2,6-di(3-alkylphenyl)pyridines, such as 2,6-di(3-methylphenyl)pyridine, 2,6-di(3-ethylphenyl)pyridine, 2,6-di(3-n-propylphenyl)pyridine, 2,6-di(3-isopropylphenyl)pyridine, 2,6-di(3-n-propylphenyl)pyridine, 2,6-di(3 tert-butylphenyl)pyridine; 2,6-di(3-arylphenyl)pyridines, such as 2,6-di(3-phenylphenyl)pyridine; 2,6-di(3-alkoxyphenyl)pyridines, such as 2,6-di(3-methoxyphenyl)pyridine and 2,6-di(3-ethoxyphenyl)pyridine; 2,6-di(2-alkylphenyl)pyridines, such as 2,6-di(2-methylphenyl)pyridine, 2,6-di(2-ethylphenyl)pyridine, 2,6-di(2-n-propylphenyl)pyridine, 2,6-di(2-isopropylphenyl)pyridine, 2,6-di(2-n-butylphenyl)pyridine, and 2,6-di(2-tert-butylphenyl)pyridine; 2,6-di(2-arylphenyl)pyridines, such as 2,6-di(2-phenylphenyl)pyridine; 2,6-di(2-alkoxyphenyl)pyridines, such as 2,6-di(2-methoxyphenyl)pyridine and 2,6-di(2-ethoxyphenyl)pyridine; and 2,6-bis disubstituted phenylpyridines, such as 2,6-bis(3,4-dimethylphenyl)pyridine, 2,6-bis(3,4-diethylphenyl)pyridine, 2,6-bis(3,5-dimethylphenyl)pyridine, 2,6-bis(3,5-diethylphenyl)pyridine, 2,6-bis(3,5-dimethoxyphenyl)pyridine, and 2,6-bis(3,5-diethoxyphenyl)pyridine. Among those preferred are 2,6-diarylpyridines whose aryl groups at the 2- and 6- positions are phenyl, phenyl having alkyl (may be branched), or phenyl having phenyl. The use of these compounds advantageously allows asymmetric Mannich reaction with relatively high yield and good enantiomeric excess. Examples of 2,6-disubstituted pyridine whose substituents are branched alkyl groups include 2,6-di-tert-butylpyridine, 2,6-di-sec-butylpyridine, 2,6-diisobutylpyridine, 2,6-diisopropylpyridine, and 2,6-di(2,4,6-mesityl)pyridine.

For the asymmetric Mannich catalyst of the present invention, the molar ratio of the 2,6-disubstituted pyridine to the optically active 1,1'-binaphthyl-2,2'-disulfonic acid compound must be set in the range of 0.75 to 3. If the molar ratio is less than 0.75, the enantiomeric excess of the optically active β-aminocarbonyl derivative is undesirably reduced. If the molar ratio is more than 3, the acidity is undesirably reduced to reduce the yield of β-aminocarbonyl derivative. In order to increase both the yield and the enantiomeric excess of β-aminocarbonyl derivative, it is preferable that the molar ratio be set in the range of 1 to 2.5.

The asymmetric Mannich catalyst of the present invention may be provided in a mixture prepared by mixing an optically active 1,1'-binaphthyl-2,2'-disulfonic acid compound and a 2,6-disubstituted pyridine in a solvent, or in a mixture prepared by evaporating the solvent from the liquid mixture. For example, if the solvent of the mixture is the same as the solvent used for the asymmetric Mannich reaction, the asymmetric Mannich catalyst may be provided in the mixture containing the solvent. If a different solvent from the solvent used for the asymmetric Mannich reaction is used, the solvent of the mixture may be evaporated and then another solvent for the asymmetric Mannich reaction may be added. The solvent used for preparing the catalyst is not particularly limited as long as it can dissolve the 1,1'-binaphthyl-2,2'-disulfonic acid compound and the 2,6-disubstituted pyridine. Examples of such a solvent include nitriles such as acetonitrile, halogenated hydrocarbons such as dichloromethane, ethers such as tetrahydrofuran, amides such as dimethylformamide, and aromatic hydrocarbons such as toluene.

### [Process for manufacturing β-aminocarbonyl derivative]

The amount of asymmetric Mannich catalyst used in the process for manufacturing a β-aminocarbonyl derivative according to the present invention is preferably, but not limited to, 0.1 to 10 mol% relative to the reaction substrate, and more preferably 1 to 5 mol%. If it is less than 0.1 mol%, the enantiomeric excess can be reduced undesirably. If it is increased to more than 10 mol%, the yield and the enantiomeric excess are not greatly increased. Such a large amount is disadvantageous in view of economical efficiency. However, some of the combinations of the reaction substrate and the asymmetric Mannich catalyst may produce good result even if the amount of asymmetric Mannich catalyst is outside such ranges.

The aldimine compound used in the manufacturing process of the β-aminocarbonyl derivative of the present invention is expressed by Ar-CH=NR¹ (Ar represents an aryl group, and R¹ represents tert-butoxycarbonyl (Boc), benzyloxycarbonyl (Cbz), or 2,2,2-trichloroethoxycarbonyl (Troc)). The aryl group in the expression is a substituted or unsubstituted aromatic hydrocarbon group. Exemplary aromatic hydrocarbon groups include phenyl, naphthyl, phenanthryl, and anthranil. If the aromatic hydrocarbon group has a substituent, examples of the substituent include alkyl, alkenyl, cycloalkyl, aryl, and alkoxy. Exemplary alkyl groups include methyl, ethyl, n-propyl, isopropyl, n-butyl, isobutyl, sec-butyl, and tert-butyl. Exemplary alkenyl groups include vinyl, allyl, butenyl, and styryl. Exemplary cycloalkyl groups include cyclopentyl and cyclohexyl. Exemplary aryl groups include phenyl, biphenyl, naphthyl, binaphthyl, and anthryl. R¹ acts as a protecting group. If Boc is introduced as the protecting group, deprotection can be performed under highly acidic conditions, such as in trifluoroacetic acid or a hydrochloric acid-ethyl acetate solution. If Cbz is used as the protecting group, deprotection can be performed by hydrogenation reaction using palladium as a catalyst, or by Birch reduction. If Troc is used, deprotection can be performed by bringing zinc powder-acetic acid into a reaction. If a benzoyl group is used as the protecting group, the enantiomeric excess is significantly reduced. The carbonyl compound used in the manufacturing process of the β-aminocarbonyl derivative of the present invention is not particularly limited as long as it is a carbonyl compound having hydrogen at the α-position. Preferably, the carbonyl compound is 1,3-dicarbonyl compound, such as 1,3-diketone, 1,3-ketoester, or 1,3-ketoamide. The 1,3-dicarbonyl compound may have cyclic ketone in its molecule. The amount of carbonyl compound to be used, which may depend on the reaction conditions, is preferably 0.67 to 1.5 equivalents to the aldimine compound.

In the manufacturing process of the β-aminocarbonyl derivative of the present invention, the reaction solvent is preferably, but not limited to, a halogenated hydrocarbon solvent, a nitrile solvent, or a cyclic ether solvent. Exemplary halogenated hydrocarbon solvents include methylene chloride, 1,1-dichloroethane, and 1,2-dichloroethane. Exemplary nitrile solvents include acetonitrile and propionitrile. Exemplary cyclic ether solvents include tetrahydrofuran (THF) and 1,4-dioxane. Among those preferred are methylene chloride, acetonitrile and THF.

In the manufacturing process of the β-aminocarbonyl derivative of the present invention, the reaction temperature is preferably, but not limited to, -40 to 50°C, and more preferably 0 to 30°C. The reaction time can be set to a period until the reaction substrate is consumed, or a period until the reaction stops, and it is set, in general, in the range of several minutes to several tens of hours.

In the manufacturing process of the (β-aminocarbonyl derivative of the present invention, contamination of the reaction system with moisture can reduce the yield and the enantiomeric excess. Accordingly, it is preferably that the reaction is conducted in the presence of a desiccant. Examples of the desiccant include magnesium sulfate, sodium sulfate, and calcium sulfate.

### Examples

### A. Process for preparing disulfonic acid

### [Example A-1]

(R)-1,1'-binaphthyl-2,2'-disulfonic acid (compound 5) was synthesized according to the flow chart shown in Chemical Formula 1. The procedure of the synthesis will be described below.

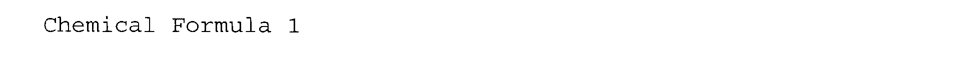

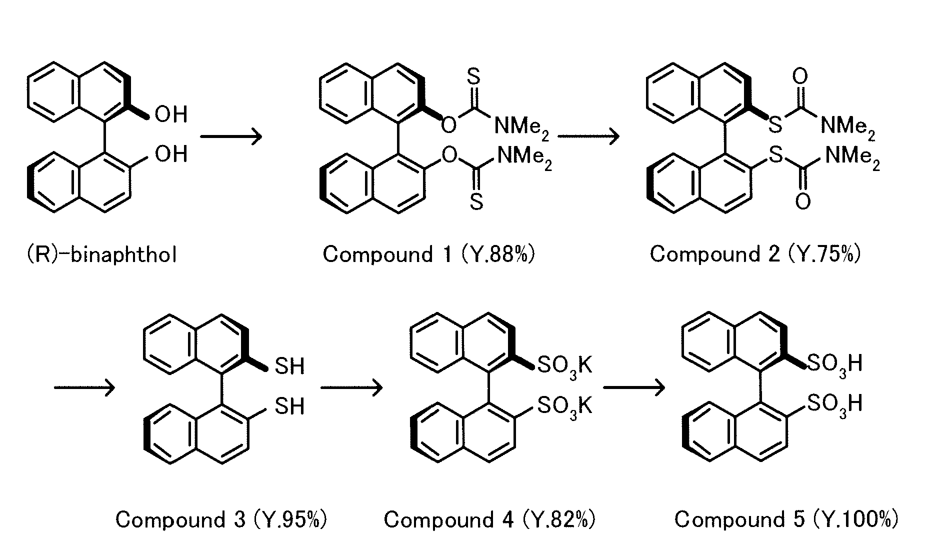

Dehydrated dimethylformamide (DMF) (100 mL) was added in a reaction vessel containing sodium hydride (4.4 g, 110 mmol, 60% oil dispersion) in a nitrogen atmosphere. The resulting suspension was cooled to 0°C and (R)-binaphthol (14.3 g, 50 mmol) was added. The mixture was heated to room temperature and stirred for 1 hour. Subsequently, dimethylthiocarbamoyl chloride (13.6 g, 110 mmol) was added and the mixture was stirred at 85°C for 2 hours. After confirming the completion of the reaction by TLC and then cooling the reaction product to room temperature, 1% by weight aqueous solution of potassium hydroxide (300 mL) was added to produce a white precipitation. The precipitation was filtered to be separated out. The separated solid was rinsed with water and dried under reduced pressure. The crude product was purified through a silica gel column chromatography (chloroform or hexane/ethyl acetate), and subsequently recrystallized (chloroform, hexane) to obtain (R)-1,1'-binaphthyl-2,2'-diyl-O,O'-bis(N,N-dimethylthiocarbamate) (compound 1) with an yield of 88% (20.2 g).

Compound 1 (8.0 g, 17.3 mmol) was placed in a reaction vessel and was exposed to microwaves of 300 W in power at 200°C for 20 minutes. After confirming the completion of the reaction by TLC, the crude product was purified through a silica gel column chromatography (chloroform or hexane/ethyl acetate) to obtain (R)-1,1'-binaphthyl-2,2'-diyl-S,S-bis(N,N-dimethylthiocarbamate) (compound 2) with an yield of 75% (5.97 g).

A reaction vessel equipped with a reflux condenser was charged with lithium aluminium hydride (0.68 g, 18 mmol) in a nitrogen atmosphere. After cooling the reaction vessel to 0°C, dehydrated tetrahydrofuran (THF) (10 mL) was added in the reaction vessel. Subsequently, the solution (10 mL) of compound 2 (1.38 g, 3.0 mmol) in THF was dropped into the reaction vessel. Then, the mixture was stirred at 0°C for 12 hours, and further stirred at 50°C for 12 hours. After confirming the completion of the reaction by TLC, the reaction vessel was cooled to 0°C, and saturated aqueous solution of sodium sulfate was carefully dropped into the vessel with violently stirring. Subsequently, the reaction mixture was stirred at 0°C for 30 minutes, and was then filtered through cerite. The residue was rinsed with diethyl ether, and the collected organic phase was evaporated under reduced pressure. The resulting concentrate was purified through a silica gel column chromatography (chloroform or hexane/ethyl acetate) to obtain (R)-1,1'-binaphthyl-2,2'-dithiol (compound 3) with an yield of 95% (0.907 g).

Hexamethylphosphoramide (HMPA)(10 mL) was added in a reaction vessel containing compound 3 (0.477 g, 1.5 mmol) and potassium hydroxide (0.504 g, 9.0 mmol). The vessel was purged with oxygen, and the reaction mixture was stirred at 80°C for 5 days under 7 atmospheres of oxygen. After being cooled to room temperature, the reaction product was purified through a silica gel column chromatography (chloroform/methanol = 1/1) to obtain potassium (R)-1,1'-binaphthyl-2,2'-disulfonate (compound 4) with an yield of 82% (0.602 g). The results of X-ray analysis of compound 4 are shown in Fig. 1, and the results of NMR analysis are shown below. The unit of NMR chemical shifts is ppm (the same applies below).

¹H NMR (300 MHz, CD₃OD) δ 6.98 (dd, J = 8.4, 0.9 Hz, 2H), 7.18 (ddd, J = 8.6, 6.9, 1.2 Hz, 2H), 7.44 (ddd, J = 8.1, 6.9, 0.9 Hz, 2H), 7.89 (d, J = 8.1 Hz, 2H), 7.99 (d, J = 8.4 Hz, 2H), 8.14 (d, J = 9.0 Hz, 2H). HRMS calcd for C₂₀H₁₃K₂O₆S₂ [M+H]⁺ 490.9428, found 490.9423.

Compound 4 (0.735 g, 1.5 mmol) dissolved in 5% methanol aqueous solution was passed through a cation exchange resin (Amberlite IR120H) (100 cm³). The solvent (water, methanol) in the collected liquid was evaporated under reduced pressure, and the remainder of the liquid was subjected to azeotropic dehydration with toluene. Then, the product was dried under reduced pressure of 1 to 2 Torrs to obtain (R)-1,1'-binaphthyl-2,2'-disulfonic acid (compound 5) with an yield of 100% (0.621 g). The spectrum data of compound 5 are shown below.

¹H NMR (300 MHz, CD₃CN) δ6.40 (br, 2H), 6.90 (d, J = 8.4 Hz, 2H), 7.27 (m, 2H),7.56 (m, 2H), 8.02 (d, J = 8.1 Hz, 2H), 8.16 (d, J = 2.1 Hz, 4H). ¹H NMR (CD₃OD, 300 MHz) δ 7.01 (d, J = 8.1 Hz, 2H), 7.18 (t, J = 7.2 Hz, 2H), 7.43 (t, J = 8.1 Hz, 2H), 7.90 (d, J = 7.2 Hz, 2H), 8.00 (d, J = 8.4 Hz, 2H), 8.14 (d, J = 8.7 Hz, 2H). HRMS calcd for C₂₀H₁₃O₆S₂ [M-H]⁻ 413.0154, found 413.0154. HRMS calcd for C₂₀H₁₄O₆S₂ [M]⁺ 414.0232, found 414.0230.

### [Example A-2]

A reaction was conducted in the same manner as in Example A-1, except that 3 equivalents of potassium hydroxide, instead of 6 equivalents of potassium hydroxide, was used to compound 3 for the oxidation of compound 3 to compound 4 in Example A-1. As a result, compound 4 was obtained with an yield of 66%.

### [Example A-3]

A reaction was conducted in the same manner as in Example A-1, except 5 atmospheres of oxygen was applied instead of 7 atmospheres of oxygen for the oxidation of compound 3 to compound 4 in Example A-1. As a result, compound 4 was obtained with an yield of 55%.

### [Comparative Example A-1]

A reaction was conducted in the same manner as in Example A-1, except that the oxidation of compound 3 to compound 4 in Example A-1 was performed under 1 atmosphere of oxygen for 8 days instead of under 7 atmospheres of oxygen for 5 days. This experiment was repeated. The yield of compound 4 was 42% in some cases, but was only less than 5% in other cases. The conditions of this oxidation were the same as the conditions of monothiol oxidation described in Tetrahedron, vol. 21, pp. 2271-2280 (1965).

### [Comparative Example A-2]

For the oxidation of compound 3 to compound 4 in Example A-1, Oxone (trade name of a product available from Du Pont) was used instead of oxygen. More specifically, compound 3 and Oxone (20 equivalents to compound 3) were added to the mixed solvent of acetonitrile and water (volume ratio 2:1) and reacted at room temperature for 24 hours. As a result, only about 10% of compound 4 was obtained.

### [Comparative Example A-3]

For the oxidation of compound 3 to Compound 4 in Example A-1, chromic acid (CrO₃) was used instead of oxygen. More specifically, compound 3 and chromic acid (10 equivalents to compound 3) were added to the mixed solvent of acetic acid and water (volume ratio 2:1) and reacted at room temperature for 14 hours. As a result, the reaction product was a mixture of many compounds, and compound 4 could not be isolated.

### [Comparative Example A-4]

For the oxidation of compound 3 to Compound 4 in Example A-1, sodium periodate (NaIO₄) was used instead of oxygen. More specifically, compound 3, sodium periodate (10 equivalents to compound 3) and ruthenium trichloride (RuCl₃) (5 mol% to compound 3) were added to the mixed solvent of carbon tetrachloride, acetonitrile and water (volume ratio 2:2:3) and reacted at room temperature for 24 hours. As a result, the reaction product was a mixture of many compounds, and compound 4 could not be isolated.

B. Process for manufacturing asymmetric Mannich catalyst and β-aminocarbonyl derivative

### [Example B-1]

An asymmetric Mannich catalyst was prepared and isolated as below (see Chemical Formula 2). More specifically, dehydrated acetonitrile (10 mL) was added to a reaction vessel containing compound 5 (0.414 g, 1 mmol) and 2,6-diphenylpyridine (0.462 g, 2 mmol) in a nitrogen atmosphere, and the resulting solution was stirred at room temperature for 2 hours. The solution was subsequently evaporated under reduced pressure and dried under a pressure reduced to 1 to 2 Torrs to obtain optically active ammonium 1 ,1'-binaphthyl-2,2'-disulfonate (compound 6) with an yield of 100% (0.87 g). The spectrum data of compound 6 are shown below.

¹H NMR (300 MHz, CD₃CN) δ 6.00 (br, 2H), 6.86 (dd, J = 8.4, 1.2 Hz, 2H), 7.23 (m, 2H), 7.48-7.62 (m, 14H), 7.92-8.23 (m, 20H). LRMS calcd for C₅₄H₄₁N₂O₆S₂ [M+H]⁺ 877, found 877.

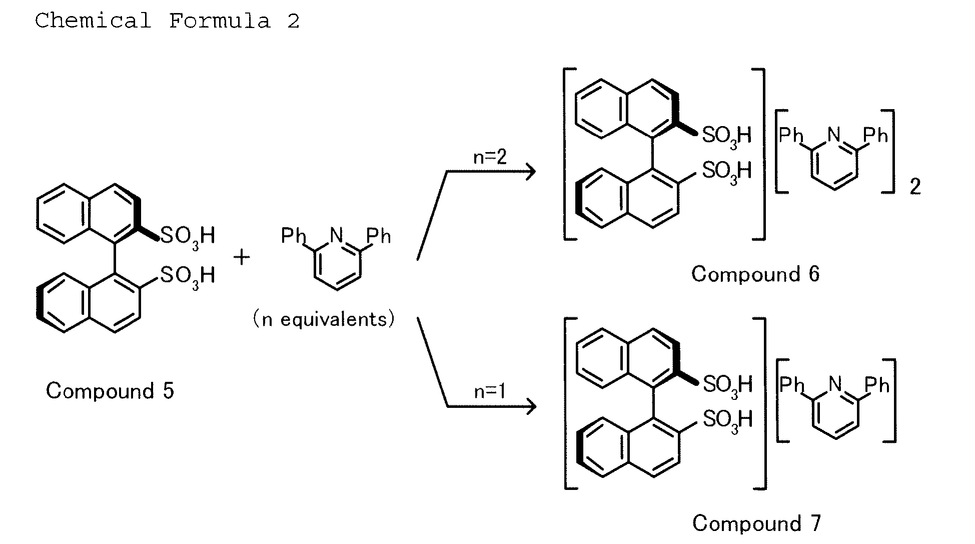

### [Example B-2]

An asymmetric Mannich catalyst (compound 7) was isolated in the same manner as in Example B-1, except that 1 mmol of 2,6-diphenylpyridine was used (see Chemical Formula 2). The spectrum data of compound 7 are shown below.

¹H NMR (300 MHz, CD₃CN) δ 6.00 (br, 2H), 6.86 (dd, J = 8.4, 1.2 Hz, 2H), 7.23 (m, 2H), 7.48-7.62 (m, 8H), 7.92-8.23 (m, 13H). LRMS calcd for C₃₇H₂₈NO₆S₂ [M+H]⁺ 646, found 646.

### [Example B-3]

A β-aminocarbonyl derivative was synthesized by an asymmetric Mannich reaction (see Chemical Formula 3). More specifically, distilled acetonitrile (2 mL) was added to a Schlenk reaction tube containing (R)-1,1'-binaphthyl-2,2'-disulfonic acid (5.2 mg, 0.0125 mmol) and 2,6-diphenylpyridine (5.8 mg, 0.025 mmol) in a nitrogen atmosphere. The reaction mixture was stirred at room temperature for 15 minutes. Then, the solvent was evaporated under reduced pressure, and the reaction product was dried at 1 to 2 Torrs for 1 hour. Subsequently, distilled CH₂Cl₂ (1.5 mL) was added, and the mixture was stirred at room temperature for 30 minutes. The resulting solution was cooled to 0°C, and benzaldehyde imine whose nitrogen is protected with Cbz (compound 8) (59.8 mg, 0.25 mmol in 0.5 mL of CH₂Cl₂) and then acetyl acetone (27.5 mg, 0.275 mmol in 0.5 mL of CH₂Cl₂) were dropped into the solution over a period of 1 hour. Then, the reaction mixture was further stirred at 0°C for 30 minutes. After confirming the completion of the reaction by TLC, saturated aqueous solution of sodium hydrogencarbonate (10 mL) was added, and the mixture was returned to room temperature. The organic phase was extracted from this solution with ethyl acetate (15 mL x 2). The extracted organic phase was rinsed with saturated aqueous solution of sodium chloride (10 mL), and was dried with anhydrous sodium sulfate. After being dried, the organic phase was filtered through cerite, and the solvent was evaporated under reduced pressure. The resulting concentrate was purified through a silica gel column chromatography (hexane/ethyl acetate = 3/1) to obtain a (β-aminocarbonyl derivative (compound 9) shown in Chemical Formula 3 with an yield of 74%. Furthermore, the enantiomeric excess of the product was determined to be 92% ee by a high performance liquid chromatography (hexane/ethanol = 9/1, 1.0 mL/min) charged with chiral column (AD-H). The spectrum data of compound 9 are shown below.

¹H NMR (300 MHz, CDCl₃) δ 2.10 (s, 3H), 2.19 (brs, 3H), 4.24 (d, J = 6.3 Hz, 1H), 5.07 (s, 2H), 5.55 (br, 1H), 6.11 (br, 1H), 7.23-7.36 (m, 10H). ¹³C NMR (75 MHz, CDCl₃) δ 30.0, 30.4, 54.2, 67.0, 71.4, 126.3 (2C), 127.8 (2C), 127.9, 128.1, 128.4 (2C), 128.8 (2C), 136.0, 139.3, 155.7, 202.2, 204.4. IR (KBr) 3362, 1730, 1692, 1530, 1254, 1026, 757, 701 cm⁻¹. [α]_{D}^{23.7} = -3.2 (c 0.5, CHCl₃). C₂₀H₂₁NNaO₄ [M+Na]⁺ 362.1368, found 362.1374. HPLC (Daicel Chiralpack AD-H, Hexane:EtOH = 9:1, flow rate = 1 mL/min) t_{R} = 47.3 min (minor, S), 52.3 min (major, R).

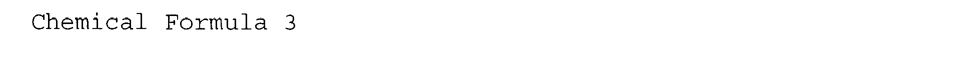

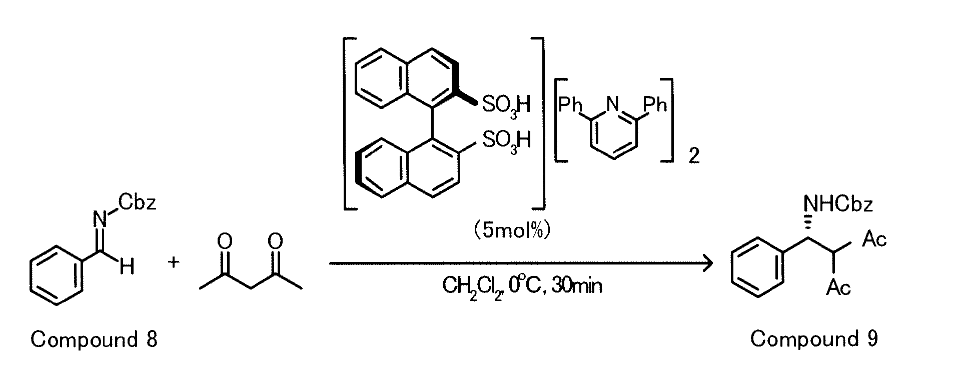

Compound 8 was synthesized according to the process described in J. Am. Chem. Soc., vol. 124, pp. 12964-12965 (2002) and J. Org. Chem., vol. 59, pp. 1238-1240 (1994). Compound 9 was converted to a methyl ester form (compound 10) according to formula (1) in Chemical Formula 4. Meanwhile, (S)-methyl ester form and (R)-methyl ester were prepared from commercially available (S)-phenylglycine and (R)-phenylglycine according to formulae (2) and (3), respectively. The resulting products were used as reference compounds. Then, compound 10 and the (S)- and (R)-methyl ester forms (reference compounds) were analyzed by a chiral high performance liquid chromatography, and compound 10 was thus determined to be in (S)-methyl ester form (92% ee). Thus, the absolute configuration of compound 9 was determined to be R-form. Chemical Formula 4 is obtained from J. Am. Chem. Soc., vol. 126, p. 5356 (2004).

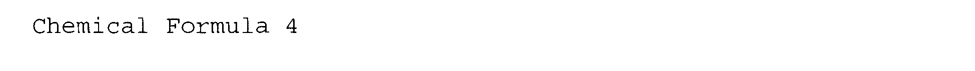

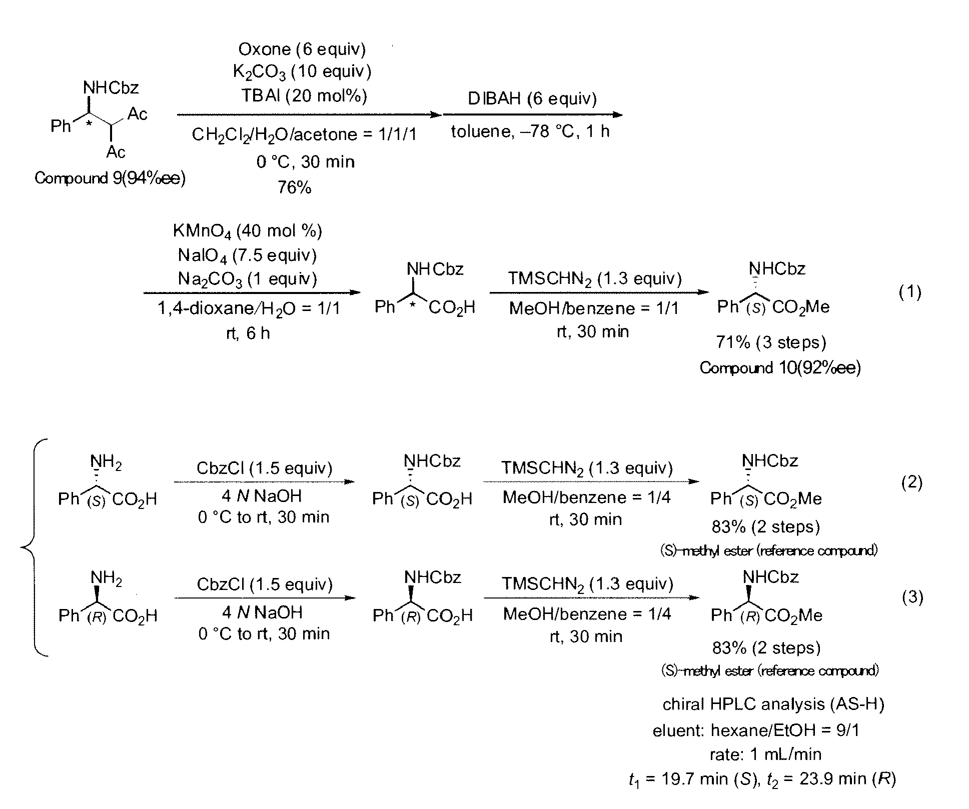

### [Example B-4]

An asymmetric Mannich reaction was performed in the same manner as in Example B-3, except that a benzaldehyde imine whose nitrogen is protected with Boc was used as an aldimine compound instead of the benzaldehyde imine (compound 8) of Example B-3 whose nitrogen is protected with Cbz. As a result, the corresponding β-aminocarbonyl derivative (compound similar to compound 9, but its nitrogen was bonded with Boc instead of Cbz) was obtained with an yield of 83% and an enantiomeric excess of 85% ee. The spectrum data of compound 11 are shown below.

¹H NMR (300 MHz, CDCl₃) δ 1.40 (s, 9H), 2.12 (s, 3H), 2.20 (brs, 3H), 4.22 (d,J = 6.6 Hz, 1H), 5.50 (br, 1H), 5.80 (br, 1H), 7.23-7.36 (m, 5H). ¹³C NMR (75 MHz, CDCl₃) δ 28.2 (3C), 30.1, 30.5, 53.7, 71.6, 80.1, 126.3 (2C), 127.7, 128.8(2C), 139.8, 155.1, 202.6, 204.7. IR (KBr) 3397, 2976, 2926, 1730, 1692, 1517,1362, 1288, 1169, 1048, 754, 704 cm⁻¹. [α]_{D}^{22.9} = +20.8 (c 0.5, CHCl₃). HRMScalcd for C₁₇H₂₃NNaO₄ [M+Na]⁺ 328.1525, found 328.1525. HPLC (Daicel Chiralpack AD-H, Hexane:EtOH = 9:1, flow rate = 1 mL/min) t_{R} = 11.5 min (minor, S), 14.8 min (major, R).

### [Example B-5]

An asymmetric Mannich reaction was performed in the same manner as in Example B-3, except that a benzaldehyde imine having whose nitrogen is protected with Troc was used as an aldimine compound instead of the benzaldehyde imine (compound 8) whose nitrogen is protected with Cbz of Example B-3, and 2.6 equivalents of 2,6-diphenylpyridine was used to 1,1'-binaphthyl-2,2'-disulfonic acid. As a result, the corresponding β-aminocarbonyl derivative (compound similar to compound 9, but its nitrogen was bonded with Troc instead of Cbz) was obtained with an yield of 87% and an enantiomeric excess of 58% ee.

### [Comparative Examples B-1 to B-3, Examples B-6 to B-10]

Although Example B-3 used 2 equivalents of 2,6-diphenylpyridine to 1,1'-binaphthyl-2,2'-disulfonic acid, the equivalent n of 2,6-diphenylpyridine was set as shown in Table 1 for the asymmetric Mannich reactions of Comparative Examples B-1 to B-3 and Examples B-6 to B-10. The results are shown in Table 1 (Table 1 includes the results of Example B-3). Table 1 clearly shows that when only 1,1'-binaphthyl-2,2'-disulfonic acid was used (Comparative Example B-1) and when the equivalent n was set to 0.25 or 0.5 (Comparative Examples B-2 and B-3), the β-aminocarbonyl derivative could be produced with a high yield, but the enantiomeric excess was extremely low. On the other hand, when the equivalent n was set in the range of 0.75 to 3 (Examples B-3 and B-6 to B-10), the β-aminocarbonyl derivative was obtained with a high yield and a high enantiomeric excess. When the equivalent n was 3 (Example B-10), however, the yield tended to be reduced. This is probably because the acidity was reduced due to the excessive amount of amine (2,6-diphenylpyridine).

**Table 1**

| | | | |
|---|---|---|---|
| 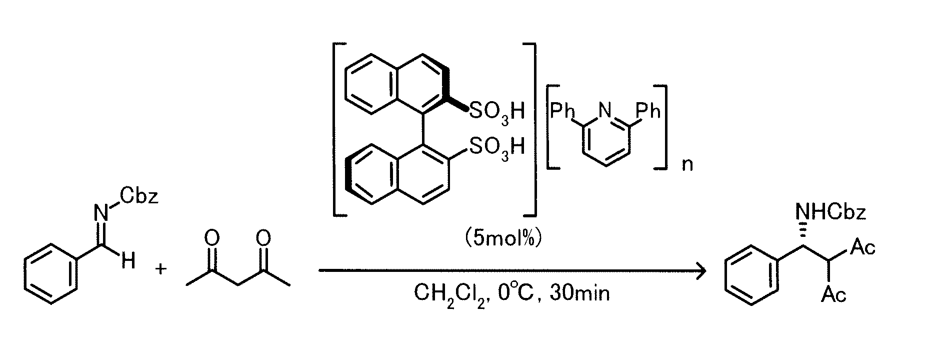 | | | |

| | n | yield | ee |
|---|---|---|---|
| Comparative Example 1 | 0 | 81% | 17%ee |
| Comparative Examples 2 | 025 | 82% | 17%ee |
| Comparative Example 3 | 0.5 | 83% | 34%ee |
| Example 6 | 0.75 | 81% | 79%ee |
| Example 7 | 1 | 82% | 84%ee |
| Example 8 | 1.5 | 84% | 90%ee |
| Example 3 | 2 | 74% | 92%ee |
| Example 9 | 2.5 | 76% | 95%ee |
| Example 10 | 3 | 68% | 86%ee |

### [Examples B-11 and B-12]

Although methylene chloride was used as the solvent of the asymmetric Mannich reaction in Example B-3, THF and acetonitrile were used in Examples B-11 and B-12, respectively. As a result, as shown in Table 2, each example produced the β-aminocarbonyl derivative with a high yield and a high enantiomeric excess.

**Table 2**

| | | | |
|---|---|---|---|
| 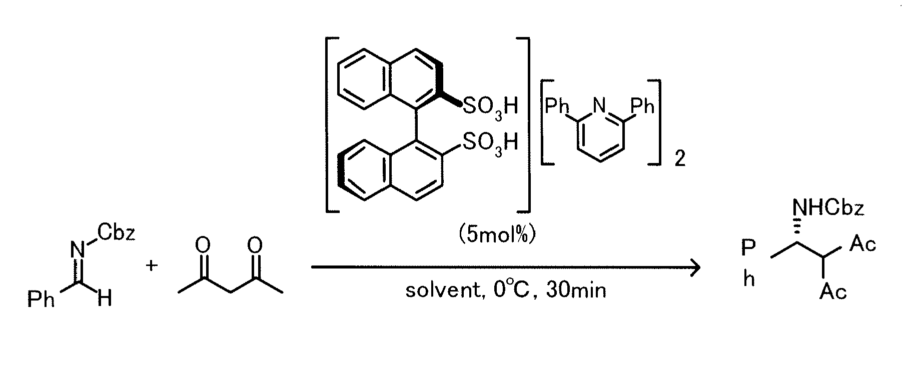 | | | |

| | solvent | yield | ee |
|---|---|---|---|
| Example 3 | CH₂Cl₂ | 74% | 92%ee |
| Example 11 | THF | 85% | 97%ee |
| Example 12 | CH₃CN | 80% | 95%ee |

### [Examples B-13 and B-21]

Example B-4 prepared the asymmetric Mannich catalyst using 1,1'-binaphthyl-2,2'-disulfonic acid and 2 equivalents of 2,6-diphenylpyridine. In Examples B-13 to B-21, asymmetric Mannich reactions were conducted using the benzaldehyde imine whose nitrogen is protected with Boc as a substrate in the same manner as in Example B-4, except that the asymmetric Mannich catalyst was prepared using several types of amine in a proportion of n equivalents as shown in Table 3. The results are shown in Table 3. Table 3 clearly shows that Examples B-13 to B-21 produced the β-aminocarbonyl derivative with high yields and that their enantiomeric excesses were good.

**Table 3**

| | | | | | |
|---|---|---|---|---|---|
| 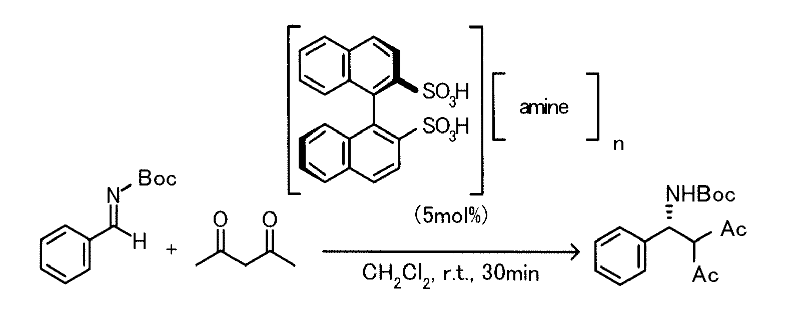 | | | | | |

| Example No. | amine | yield [ee] | Example No. | amine | yield [ee] |
|---|---|---|---|---|---|
| 13 | 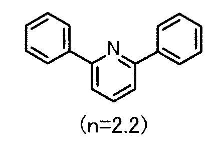 | 95% [70%ee] | 18 | 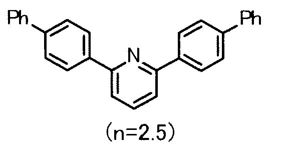 | 93% [60%ee] |
| 14 | 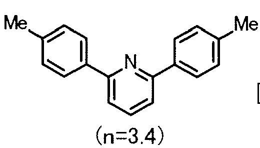 | 93% [73%ee] | 19 | 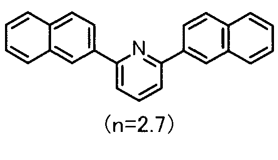 | 86% [48%ee] |
| 15 | 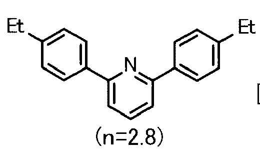 | 91% [61%ee] | 20 | 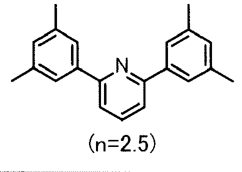 | 92% [61%ee] |
| 16 | 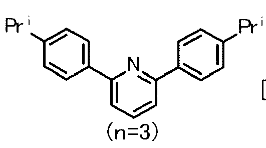 | 89% [57%ee] | 21 | 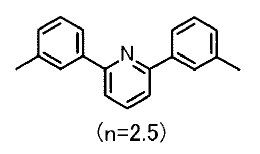 | 92% [53%ee] |
| 17 | 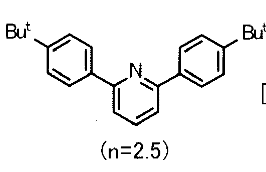 | 85% [56%ee] | | | |

### [Example B-22]

An asymmetric Mannich reaction using a benzaldehyde imine having whose nitrogen is protected with Cbz as a substrate was conducted in the same manner as in Example B-3, except that methyl acetoacetate was used instead of acetyl acetone of Example B-3 (see Chemical Formula 5). As a result, the corresponding β-minocarbonyl derivative was obtained with an yield of 58%. The diastereomer ratio (dr) and the enantiomeric excess (ee) of this reaction are shown in Chemical Formula 5.

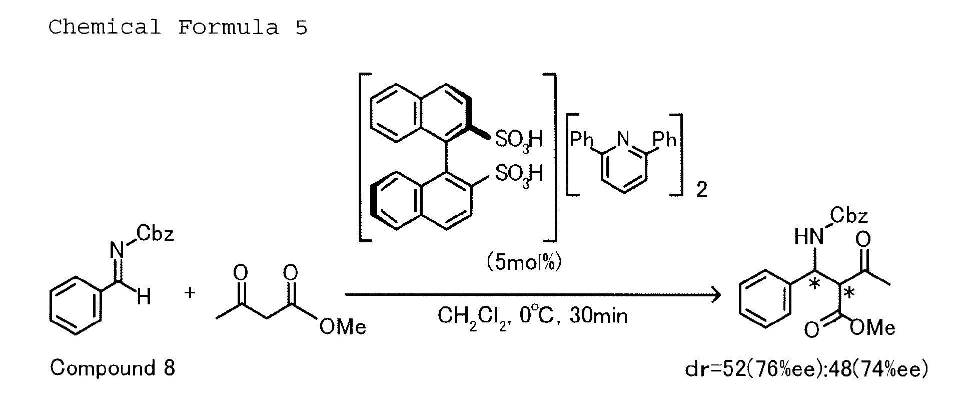

### [Example B-23]

(R)-1,1'-binaphthyl-2,2'-disulfonic acid (0.0025 mmol) and 2,6-diphenylpyridine (0.005 mmol) were stirred in acetonitrile, and then the solvent was evaporated under reduced pressure. Subsequently, magnesium sulfate (0.42 mmol) and distilled CH₂Cl₂ were added, and the mixture was stirred at room temperature for 30 minutes. The resulting solution was cooled to 0°C, and benzaldehyde imine whose nitrogen is protected with Cbz (0.375 mmol in 0.5 mL of CH₂Cl₂) and then acetyl acetone (0.25 mmol in 0.5 mL of CH₂Cl₂) were dropped into the solution over a period of 1 hour. Then, the reaction mixture was further stirred at 0°C for 30 minutes. As a result, the corresponding β-aminocarbonyl derivative was obtained with an yield of 91% and an enantiomeric excess of 90% ee.

### [Examples B-24 to B-32]

Reactions of Examples B-24 to B-32 were conducted according to Example B-23. More specifically, asymmetric Mannich reactions were conducted using several combinations of substrates (aldimine compound and carbonyl compound) shown in Table 4. The results are shown in Table 4 (Table 4 includes the result of Example B-23). The yield was calculated with respect to the carbonyl compound. Table 4 clearly shows that each Example produced corresponding β-aminocarbonyl derivatives with high yields and, in addition, with high enantiomeric excesses.

**Table 4**

| | | | | |
|---|---|---|---|---|
| 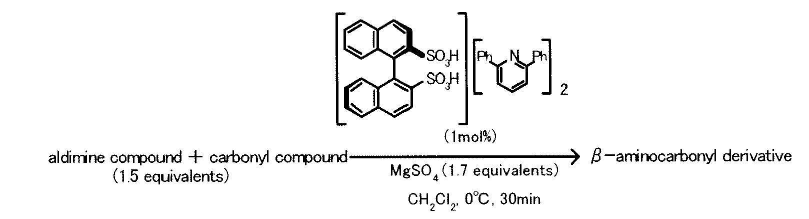 | | | | |

| Example No. | aldimine compound | carbonyl compound | β-aminocarbonyl derivative | yield [ee] |
|---|---|---|---|---|
| 23 | 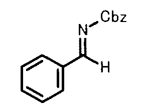 | 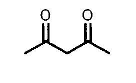 | 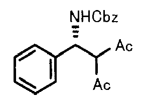 | 91% [90%ee] |
| 24 | 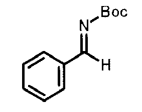 | 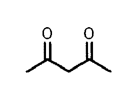 | 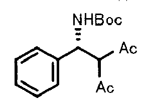 | 99% [84%ee] |
| 25 | 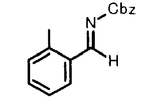 | 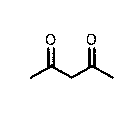 | 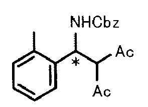 | 99% [96%ee] |
| 26 | 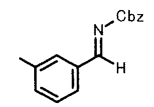 | 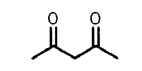 | 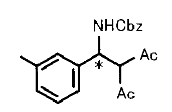 | 99% [89%ee] |
| 27 | 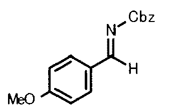 | 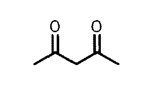 | 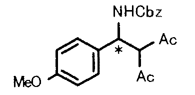 | 95% [96%ee] |
| 28 | 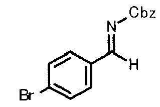 | 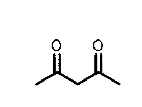 | 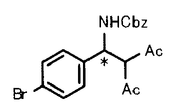 | 92% [98%ee] |
| 29 | 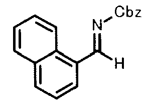 | 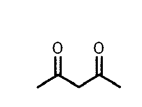 | 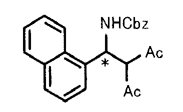 | 99% [96%ee] |
| 30 | 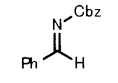 | 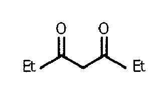 | 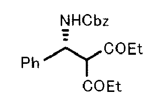 | 95% [95%ee] |
| 31 | 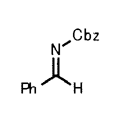 | 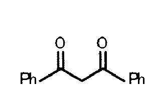 | 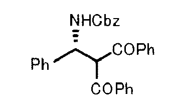 | >99% [84%ee] |
| 32 | 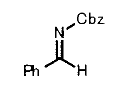 | 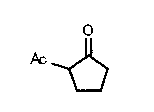 | 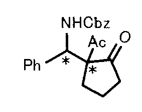 | 98% [dr = 83 : 17 (91%ee)(96%ee)] |

### [Example B-33]

An asymmetric Mannich reaction was conducted in the same manner as in Example B-3, except that 2,6-di-tert-butylpyridine was used instead of 2,6-diphenylpyridine of Example B-3. Consequently, the corresponding β-aminocarbonyl derivative (compound 9) was obtained with an yield of 32% and an enantiomeric excess of 76% ee.

### [Examples B-34 and B-35]

As shown in the formula shown together in Table 5, benzaldehyde imine (compound 8) and an oxazolidinone derivative (ketoamide, compound 12) were allowed to react with each other. The former was used in a proportion of 1.5 equivalents to the latter. Example B-34 used (R)-1,1'-binaphthyl-2,2'-disulfonic acid (5 mol%) and 2,6-diphenylpyridine (10 mmol%), and Example B-35 used (R)-1,1'-binaphthyl-2,2'-disulfonic acid (5 mol%) and 2,6-di(2,4,6-mesityl)pyridine (10 mmol%). The results are shown in Table 5.

**Table 5**

| | | | |
|---|---|---|---|
| 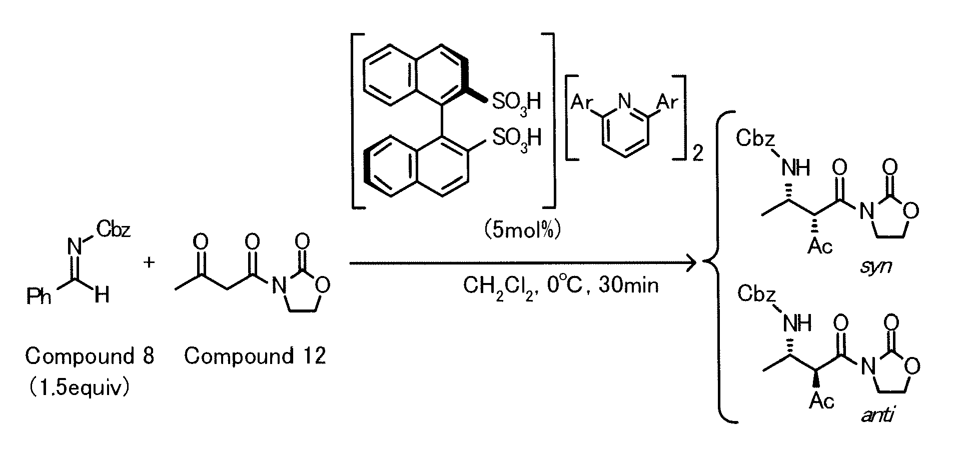 | | | |

| | Ar | yield | ee |
|---|---|---|---|
| Example 35 | Ph | 86% (dr=53:47) | 72%ee(syn) 20%ee(anti) |
| Example 36 | 2,4,6-mesityl | 81% (dr=60:40) | 93%ee(syn) 90%ee(anti) |

The present invention claims priority from the Japanese Patent Application No. 2007-276589 filed on October 24, 2007, and the Japanese Patent Application No. 2007-276590 filed on October 24, 2007, the entire cotnents of both of which are incorporated herein by reference.

### Industrial Applicability

The 1,1'-diaryl-2,2'-disulfonic acid produced by the process for manufacturing a disulfonic acid compound of the present invention can be used as, for example, an agent for introducing an asymmetric auxiliary group to an optically active amine, and an intermediate in the synthesis of an asymmetric Mannich catalyst. The asymmetric Mannich catalyst of the present invention can be applied mainly to the chemical industry. For example, the catalyst can be used in manufacture of various β-aminocarbonyl compounds, which are used, for example, as intermediates of pharmaceuticals, agricultural chemicals and cosmetics.

## Claims

1. A process for manufacturing a disulfonic acid compound,
wherein an axially asymmetric, optically active 1,1'-biaryl-2,2'-dithiol compound is oxidized with pressurized oxygen in the presence of a strong base, thereby producing a salt of a corresponding optically active disulfonic acid compound.

2. The process for manufacturing the disulfonic acid compound according to Claim 1,
wherein the strong base is an alkali metal hydroxide and is used in a proportion of 3 equivalents or more to the dithiol compound.

3. The process for manufacturing the disulfonic acid compound according to Claim 1 or 2,
wherein the pressurized oxygen has a pressure of 5 atmospheres or more.

4. The process for manufacturing the disulfonic acid compound according to any one of Claims 1 to 3,
wherein the 1,1'-biaryl-2,2'-dithiol compound is oxidized in a polar aprotic solvent.

5. The process for manufacturing the disulfonic acid compound according to any one of Claims 1 to 4,
wherein the reaction temperature is 50 to 100°C.

6. The process for manufacturing the disulfonic acid compound according to any one of Claims 1 to 5,
wherein the salt of the disulfonic acid compound is purified through a column, and subsequently converted to a free form of the disulfonic acid compound through a cation exchange resin.

7. The process for manufacturing the disulfonic acid compound according to any one of Claims 1 to 6,
wherein the 1,1'-biaryl-2,2'-dithiol compound is a 1,1'-binaphthyl-2,2'-dithiol compound.

8. An asymmetric Mannich catalyst
comprising a mixture of an optically active 1,1'-binaphthyl-2,2'-disulfonic acid compound and a 2,6-disubstituted pyridine whose substituent is aryl or branched alkyl in a molar ratio of 1:0.75 to 1:3.

9. The asymmetric Mannich catalyst according to Claim 8,
wherein the molar ratio is 1:1 to 1:2.5.

10. The asymmetric Mannich catalyst according to Claim 8 or 9,
wherein the substituents at the 2- and 6-positions of the 2,6-disubstituted pyridine are phenyl, phenyl having branched or unbranched alkyl, or phenyl having phenyl.

11. A process for manufacturing a β-aminocarbonyl derivative,
wherein an optically active β-aminocarbonyl derivative is produced by a Mannich reaction between an aldimine compound expressed by Ar-CH=NR1 (Ar represents aryl, R1 represents tert-butoxycarbonyl (Boc), benzyloxycarbonyl (Cbz) or 2,2,2-trichloroethoxycarbonyl (Troc)) and a carbonyl compound in the presence of the asymmetric Mannich catalyst as set forth in any one of Claims 8 to 10.

12. The process for manufacturing the β-aminocarbonyl derivative according to Claim 11,
wherein the carbonyl compound is 1,3-diketone or 1,3-ketoester.

13. The process for manufacturing the β-aminocarbonyl derivative according to Claim 11 or 12,
wherein the Mannich reaction uses a halogenated hydrocarbon solvent, a nitrile solvent or a cyclic ether solvent as a reaction solvent.

14. The process for manufacturing the β-aminocarbonyl derivative according to any one of Claims 11 to 13,
wherein the Mannich reaction is performed at a reaction temperature of -40 to 50°C.

15. The process for manufacturing the β-aminocarbonyl derivative according to any one of Claims 11 to 14,
wherein the Mannich reaction is performed in the presence of a desiccant.

16. A novel disulfonate
being a salt of an optically active 1,1'-binaphthyl-2,2'-disulfonic acid compound and a 2,6-disubstituted pyridine whose substituent is aryl or branched alkyl.
